(19) Europäisches Patentamt
European Patent Office
Office européen des brevets

(11) EP 3 865 498 B1

# (12) EUROPEAN PATENT SPECIFICATION

(45) Date of publication and mention of the grant of the patent:
**25.03.2026 Bulletin 2026/13**

(21) Application number: **19890729.7**

(22) Date of filing: **29.11.2019**

(51) International Patent Classification (IPC):
***C07H 3/02*** *(2006.01)* ***C07H 1/00*** *(2006.01)*
***A23L 27/30*** *(2016.01)*

(52) Cooperative Patent Classification (CPC):
**A23L 29/30; C07H 1/00; C07H 3/02**

(86) International application number:
**PCT/KR2019/016668**

(87) International publication number:
**WO 2020/111851 (04.06.2020 Gazette 2020/23)**

## (54) D-PSICOSE CRYSTAL AND PREPARATION METHOD THEREFOR

D-PSICOSE-KRISTALL UND VERFAHREN ZU SEINER HERSTELLUNG

CRISTAL DE D-PSICOSE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2018 KR 20180152876**

(43) Date of publication of application:
**18.08.2021 Bulletin 2021/33**

(73) Proprietor: **CJ Cheiljedang Corporation**
**Seoul 04560 (KR)**

(72) Inventors:
- **PARK, Young Soo**
**Seoul 04560 (KR)**
- **LEE, Joo Hang**
**Seoul 04560 (KR)**
- **KIM, Seong Bo**
**Seoul 04560 (KR)**
- **PARK, Seung Won**
**Seoul 04560 (KR)**

(74) Representative: **Cabinet Beau de Loménie**
**103, rue de Grenelle**
**75340 Paris Cedex 07 (FR)**

(56) References cited:
**EP-A1- 3 210 478**
**FR-A1- 3 061 413**
**JP-A- 2005 263 670**
**JP-B2- 4 873 493**
**KR-A- 20110 108 185**
**KR-A- 20160 046 143**
**KR-A- 20160 062 349**
**US-A1- 2011 237 790**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 865 498 B1

## Description

### Technical Field

The present disclosure relates to a D-psicose crystal and to a method for preparing a D-psicose crystal with high a yield from a D-psicose-containing solution.

### Background Art

An epimer of D-fructose, D-psicose is known as a rare saccharide and is a type of functional saccharide, and has been known to have a caloric value of almost zero even while exhibiting a high sweetness degree of about 60% to 70% that of sugar, making it effective in the prevention and improvement of diabetes. Further, D-psicose is known to have excellent solubility, and is a material which has received attention for utilization in foods. Epimerization reaction solutions containing produced D-psicose contain a D-psicose solid content of about 20% (w/w) to 30% (w/w) with low purity, and in order to prepare a D-psicose crystal formulation of 98% or higher therefrom, separation and purification techniques such as chromatography and crystallization techniques are required. In general, methods for crystallizing saccharides use the principle of inducing the growth of a crystal within a metastable zone (the range between the saturated concentration and the lowest supersaturated concentration at which crystals spontaneously precipitate) in a supersaturated state. D-Psicose has a characteristic of exhibiting almost no change in crystal generation and crystal growth rates even in this supersaturated concentration range, and thus it may be classified as a saccharide with unfavorable particle size growth crystallization conditions.

At concentrations in the metastable zone, crystallization phenomena such as crystal nucleation do not occur, but when a new crystal is added from the outside, crystal growth occurs and crystal size increases. That is, in order to generate crystals, when a seed is added to a solution at or above its saturated concentration, the seed grows in the metastable zone, resulting in crystal growth.

Since D-psicose has a significantly lower crystal growth rate than sucrose, cooling crystallization methods are generally applied, and the development of a highly economical technology is required for industrialization.

A method of using a large amount of ethanol in a process of crystallizing the D-psicose has been reported (Kei T, et al., J. Biosci. Bioeng., 90(4), 453-455, 2000), but according to this method, although crystal nucleation occurs, crystal growth does not. Specifically, in the crystallization process, a blocking phenomenon occurs due to amorphous lumps, causing the generation of particle crystals. When such blocking occurs during the process, it becomes difficult to transfer the crystals to the outside of the crystallizer, and furthermore, pulverization cannot be performed, thereby lowering the production yield. Additionally, in the saccharide crystallization industry, the size of crystal particles is generally known to be an important factor, and when crystals generated in a mass-production system are particles, the crystals are easily removed from crystal centrifuge equipment together with the crystal mother liquor. The remaining particles in the separation agglomerate between the crystals during drying and are excluded in a sieving process, and thus the final product packaging volume decreases or marketability suffers. Accordingly, such particle crystals are not suitable for mass-production methods. Further, in the crystallization of saccharides using ethanol, when the concentration of the final remaining ethanol is high, the occurrence of bad taste and smell lower the marketability of saccharide crystal products.

EP-A-3 210 478 describes a method for producing D-psicose crystals having a purity of 98% (w/w) or more, comprising: removing impurities from a D-psicose solution to obtain a purified D-psicose solution; concentrating the purified D-psicose solution; cooling the concentrated D-psicose solution to 30°C to 40°C; seed crystallizing the D-psicose solution at 30°C to 40°C to obtain a seed crystallized massecuite; and full-scale crystallizing the seed crystallized massecuite. US 2011/237790 describes a method of producing D-psicose crystals, comprising: removing impurities from a D-psicose solution to obtain a purified D-psicose solution; concentrating the purified D-psicose solution; and crystallizing D-psicose from the concentrated D-psicose solution in a supersaturated state under a metastable zone. FR-A-3 061413 describes a process for manufacturing D-allulose crystals comprising: supplying a composition rich in D-allulose, concentrating said solution to form a mother solution to be crystallised, crystallising the mother solution to form D-allulose crystals and mother liquors.

In light of the present situation, the present inventors have found that by using a concentrated D-psicose solution and an organic solvent in an operable range, the recovery rate of the crystals may be increased and the crystal particles may be grown, and have developed a method for preparing high-purity D-psicose with a purity of 98% (w/w) or higher with no bad taste/smell, which has a crystal particle size of MA 200 or more with improved flowability in a crystallization process and improved marketability as a product.

### Disclosure

## Technical Problem

An object of the present disclosure is to provide a D-psicose crystal containing 98% (w/w) or more D-psicose and 0.001% (w/w) to 0.05% (w/w) ethanol based on 100% (w/w) of the entire crystal.

Another object of the present disclosure is to provide a preparation method for a D-psicose crystal including a first step of mixing a D-psicose-containing solution and ethanol, wherein the mixture has a water : ethanol ratio of 1:0.5 or more; and a second step of adding a seed to the mixed solution according to the first step and then cooling the same to obtain a massecuite containing the D-psicose crystal.

## Technical Solution

Hereinafter, the present disclosure will be described in more detail.

Meanwhile, each description and embodiment disclosed in the present disclosure can also be applied to each other description and embodiment. That is, all combinations of the various components disclosed in the present disclosure belong to the scope of the present disclosure. In addition, the specific description below may not limit the scope of the present disclosure.

Further, those skilled in the art may recognize or determine a plurality of equivalents to specific embodiments of the present disclosure described herein using only common experiments. In addition, such equivalents are intended to be included in the present disclosure.

As an aspect of the present disclosure to solve the objects, the present disclosure provides a D-psicose crystal containing 98% (w/w) or more D-psicose and 0.001% (w/w) to 0.05% (w/w) ethanol based on 100% (w/w) of the entire crystal.

In the present disclosure, the "D-psicose" is a monosaccharide of low caloric content, and has a molecular formula of $C_6H_{12}O_6$ and the following structural formula. In addition to a chain-like structure, hexagonal ring-like structures of an alpha type and a beta type are also included in the scope of the D-psicose.

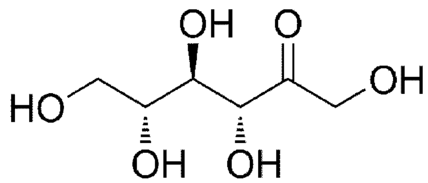

In the present disclosure, the "D-psicose crystal" is a solid in which D-psicose molecules are arranged in a regularly repeating structure, which is different from an amorphous solid lump with no repeating structure. The D-psicose crystal of the present disclosure may have a purity of 98% (w/w) or higher, specifically 98.5% (w/w) or higher, and more specifically 99% (w/w) or higher.

In the preparation of the D-psicose crystal of the present disclosure, as the addition of ethanol is included, the D-psicose crystal may contain a minute amount of ethanol. The content of the ethanol is 0.05% (w/w) or lower, specifically 0.04% (w/w) or lower, and more specifically 0.03% (w/w) or lower. The ethanol content is, for example, 0.001% (w/w) to 0.05% (w/w), 0.001% (w/w) to 0.04% (w/w), 0.001% (w/w) to 0.03% (w/w), 0.005% (w/w) to 0.05% (w/w), 0.005% (w/w) to 0.04% (w/w), 0.005% (w/w) to 0.03% (w/w), 0.01% (w/w) to 0.05% (w/w), 0.01% (w/w) to 0.04% (w/w), 0.01% (w/w) to 0.03% (w/w), 0.02% (w/w) to 0.05% (w/w), 0.02% (w/w) to 0.04% (w/w), or 0.02% (w/w) to 0.03% (w/w). According to an unlimited embodiment of the present disclosure, when the ethanol is contained at the above content, a bad taste/smell is not exhibited, and thus the D-psicose crystal may be used equivalently to a D-psicose crystal in which ethanol has not been added (Test Example 2). Since a minute amount of ethanol is contained, the surface of the D-psicose crystal may be smooth, and the D-psicose crystal may be glossy or lustrous as compared with the D-psicose crystal in which ethanol has not been added (Test Example 3). Further, when a minute amount of ethanol is contained, the fluidity of the D-psicose crystal is increased, and a high yield and degree of crystallinity can be achieved in accordance with crystallization when seeds are dispersed. The fluidity of the crystal may be confirmed by the measurement of the fluidity and a repose angle, wherein the repose angle means a maximum inclined angle at which deposits that have not yet set may be deposited without flowing down when deposited on a slope. The repose angle may be obtained by measuring an angle of the top surface of a composition with a graduator after a D-psicose crystal composition passes through a funnel installed on a horizontal surface at a predetermined speed (FIG. 6).

The % (w/w) may be used in combination with wt%. The % (w/w) may mean a crystal weight based on 100 parts by weight of the entire crystal, or a crystal weight based on 100 parts by weight of a solution containing a crystal. Specifically, the % (w/w) may mean a weight of a D-psicose crystal based on 100 parts by weight of the entire crystal, or a weight of a D-psicose crystal based on 100 parts by weight of a solution containing a crystal. The crystal may contain a dry solid (DS).

Further, the term "mean crystal particle size" of the present disclosure is a measure representing a mean size of the crystal. A method of measuring the crystal particle size of D-psicose is not limited, and methods generally used in the art may be used. As an unlimited example of measuring the crystal particle size, a comparison method (FGC), a cutting method (FGI), a flat capacity method (FGP), and the like are included.

The D-psicose crystal of the present disclosure may have a mean particle size (MA: Mean diameter of the Area distribution) of the crystal of 60 μm or more, 100 μm or more, 150 μm or more, 200 μm or more, 230 μm or more, 250 μm or more, 300 μm or more, or 320 μm or more. For example, the mean particle size may be 100 μm to 500 μm, 150 μm to 450 μm, 200 μm to 400 μm, 230 μm to 400 μm, 300 μm to 400 μm, 320 μm to 400 μm, or 320 μm to 390 μm.

The d10 of the D-psicose crystal of the present disclosure is a value corresponding to a lower 10% of the crystal distribution and may be 100 μm or more, 110 μm or more, 130 μm or more, or 150 μm or more. For example, the d10 of the D-psicose crystal may be 100 μm to 200 μm, 100 μm to 180 μm, 110 μm to 180 μm, 110 μm to 160 μm, 130 μm to 180 μm, 130 μm to 160 μm, 150 μm to 180 μm, or 150 μm to 160 μm.

The d50 of the D-psicose crystal of the present disclosure is a value corresponding to a lower 50% of the crystal distribution and may be used in combination with a median, and may be 100 μm or more, 150 μm or more, 200 μm or more, 230 μm or more, 250 μm or more, 300 μm or more, or 310 μm or more. For example, the d50 of the D-psicose crystal may be 100 μm to 500 μm, 150 μm to 450 μm, 200 μm to 400 μm, 230 μm to 400 μm, 300 μm to 400 μm, 320 μm to 400 μm, or 310 μm to 390 μm.

The d90 of the D-psicose crystal of the present disclosure is a value corresponding to a lower 90% of the crystal distribution and may be 200 μm or more, 250 μm or more, 300 μm or more, 330 μm or more, 350 μm or more, 400 μm or more, or 500 μm or more. For example, the d90 of the D-psicose crystal may be 200 μm to 800 μm, 250 μm to 700 μm, 300 μm to 650 um, 330 μm to 600 μm, 350 μm to 600 μm, 400 μm to 600 μm, or 500 μm to 590 μm.

The particle size distribution of the D-psicose crystal of the present disclosure may be confirmed by a relative standard deviation or a relative particle size distribution.

The relative standard deviation is a percentage value obtained by dividing a standard deviation by a mean particle size and may be 30% to 60%, 35% to 55%, 37% to 50%, 38% to 48%, or 40% to 46%.

The relative particle size distribution is a value obtained by dividing d50 by the difference between d90 and d10 and may be 0.8 to 1.5, 0.9 to 1.4, or 1.0 to 1.3.

As a result, an amount of the D-psicose crystal lost together with a mother liquor is decreased in the crystal centrifugal equipment to reduce the aggregation between the crystals. Accordingly, the D-psicose crystal of the present disclosure having the particle size is suitable for mass production.

As another aspect of the present disclosure to solve the object, the present disclosure provides a preparation method for a D-psicose crystal including a first step of concentrating a D-psicose-containing solution; a second step of adding a seed to the mixed solution according to the first step and then cooling the same to obtain a massecuite containing the D-psicose crystal.

The "D-psicose-containing solution" is not limited so long as it is any solution in which D-psicose is dissolved or dispersed. The D-psicose-containing solution may be a D-psicose-containing solution with a high purity of 90% (w/w) or higher, specifically 91% (w/w) or higher, 92% (w/w) or higher, 93% (w/w) or higher, 94% (w/w) or higher, or 95% (w/w) or higher, but the present disclosure is not limited thereto.

The D-psicose-containing solution may be a solution obtained through an epimerization reaction by treating a substrate for the production of D-psicose with an enzyme, or may be obtained by separating or purifying the solution. As examples which are not limited to the substrate and the enzyme, fructose and D-psicose epimerases are included, and fructose-6-phosphate, D-psicose-6-phosphate epimerase, and D-psicose-6-phosphate phosphatase are included. Specifically, the D-psicose-containing solution may be obtained by purifying a solution obtained by an epimerization reaction between fructose and D-psicose. Specifically, the fructose used as a substrate for the epimerization reaction may be dissolved and used in water at a concentration of 30 brix(%) to 50 brix(%) at a temperature of 30°C to 40°C. In the present disclosure, brix(%) means a percentage of a weight of D-psicose or fructose based on a weight of the entire solution. Here, the fructose may then be mixed with a fructose-containing solution separated by chromatography and may be used at a concentration of 30 brix(%) to 50 brix(%) at a temperature of 30°C to 40°C. Here, the fructose-containing solution separated by chromatography may use a fructose-containing fraction with a purity of 70% (w/w) or higher, specifically 75% (w/w).

The D-psicose epimerization reaction may be to produce D-psicose by epimerizing fructose in the presence of psicose epimerase, variants thereof, a strain for producing the enzyme, or a culture thereof. The D-psicose epimerase which may be used in the present disclosure may be an enzyme or a variant derived from various donor microorganisms such as *Agrobacterium tumefaciens, Flavonifractorplauti,* and *Clostridium hylemonae.* The strain for transformation is *Escherichia coli, Corynebacterium, Bacillus,* and *Aspergillus,* but is not limited only to these strains. As the strains transformed to *Escherichia coli,* for example, BL21(DE3)/pET24-ATPE [Korea Patent Publication No. 10-211-0035805], BL21(DE3)/pET24-ATPE-2 [Korea Patent Registration No. 10-1203856], *etc.* are included. The *Corynebacterium* strains include *Corynebacterium glutamicum* ATCC13032/pCJ-1-ATPE [Accession No. KCCM11046 of Korea Patent Publication

No. 10-2011-0035805], *Corynebacterium glutamicum* ATCC13032/pFIS-1-ATPE-2 [Accession No. KCCM11204P of Korea Patent Registration No. 10-1203856], *Corynebacterium glutamicum* CJ KY [Accession No. KCCM11403P of Korea Patent Registration No. 10-1455759], *Corynebacterium glutamicum* ATCC13032/pFIS-2-ATPE-2 [Accession No. KCCM11678P of Korea Patent Application No. 10-2015-0047111], *etc.,* but the present disclosure is not limited thereto.

As an example, the epimerization reaction may be performed by immobilizing psicose epimerase, a variant thereof, a strain for producing the enzyme, or a culture thereof on a carrier, for example, sodium alginate, filling isomerization reaction equipment, for example, a column, with the immobilized enzyme, and then supplying a fructose-containing solution to the filled column. A temperature in the equipment may be kept at a temperature of 40°C to 70°C, for example, 40°C to 55°C, for the epimerization reaction. At this time, the temperature of the solution containing the supplied fructose rises at, for example, 5°C to 20°C per hour, to a temperature of 40°C to 60°C, for example, 50°C, by way of a heat exchanger, and the solution passes at a space velocity (SV) [flow rate (L)/time (Hr)/resin amount (L)] of 0.5 to 3. The purity of the D-psicose produced by the epimerization reaction may be about 15% (w/w) to about 35% (w/w), for example, about 20% (w/w) to about 30% (w/w).

The solution containing the epimerized D-psicose may be cooled. The cooling is performed at the temperature of the solution or an ambient temperature in a range of 25°C to 45°C, specifically 30°C to 40°C. Specifically, the cooling may be slowly performed at 1°C to 10°C per hour, and a heat exchanger may be used in the cooling.

The D-psicose-containing solution may be obtained by separating and/or purifying a crude solution containing D-psicose. That is, the preparation method for the D-psicose crystal of the present disclosure may include separating and/or purifying a crude solution containing D-psicose before the first step.

The D-psicose-containing solution may be obtained by purifying the crude solution containing D-psicose. Specifically, the purification may be performed by at least one selected from the group consisting of decoloration by passing through a column filled with a decolorant, desalinization by ion-exchange resin chromatography, and continuous chromatography.

Specifically, the solution containing the epimerized D-psicose may be cooled and then decolored by passing through a column filled with a decolorant, and purified through a column filled with a strongly acidic cation-exchange resin and a weakly basic anion-exchange resin. In the case of using a strongly basic anion-exchange resin, the D-psicose is denatured even at a low temperature of 25°C to 45°C, and thus the purity may be deteriorated. As a result, in order to prepare D-psicose with a high yield, the weakly basic anion-exchange resin may be used, and more specifically, a 100% weakly basic anion-exchange resin may be used. For the effective removal of ionic components, the cation-exchange resin and the anion-exchange resin may be used at the same time. In this case, a ratio of the cation-exchange resin and the anion-exchange resin may be 1:0.5 to 1:3. During the ion purification, a temperature of 25°C to 45°C, specifically 30°C to 40°C is kept to prevent the denaturation of D-psicose. As a result, ionic components contained as impurities in the D-psicose-containing solution may be removed. After the ion purification, the content of the ionic components may be 20 microsiemens or less, specifically 10 microsiemens or less per unit cm when measured by an electric conduction system. The purity of the D-psicose in the ion-purified solution may be about 10% (w/w) to about 35% (w/w). Here, in the cooling and the ion purification, ethanol is removed from the mother liquor produced in a subsequent D-psicose crystallization step by distillation, and the mother liquor may then be reused. The purified D-psicose-containing solution may be concentrated and cooled.

The concentration is a process of concentrating the ion-purified D-psicose-containing solution at a D-psicose concentration in a range of 50 brix(%) to 70 brix(%), for example, 55 brix(%) to 65 brix(%). Specifically, the concentration may be performed at a temperature of 50°C to 80°C, for example, 55°C to 70°C. At this time, in the concentration, a low-temperature evaporator may be used to prevent the denaturation of the D-psicose. After the concentration, the cooling may be performed. When the cooling is performed, the cooling may be performed so that the solution or ambient temperature reaches a temperature lower than the temperature during the concentration by at least 10°C. For example, the cooling temperature may be in a range of 40°C to 60°C. The cooling may be slowly performed at a cooling rate of 5°C to 25°C per hour, and a heat exchanger may be used in the cooling. Here, in the concentration and the cooling, ethanol is removed from the mother liquor produced in a subsequent D-psicose crystallization step by distillation, and the mother liquor may then be reused.

The D-psicose-containing solution may be a solution obtained by separating fructose from the solution containing D-psicose. Specifically, the fructose-containing solution may be separated by chromatography.

The chromatography is to separate D-psicose using a difference in a weak binding force between D-psicose and metal ions attached to the ionic resin, and for example, continuous chromatography may be used. The ionic resin used for the chromatography may be a strongly acidic cation-exchange resin with K, Na, Ca, and Mg residues attached. Specifically, the ionic resin may be an ionic resin capable of separating D-psicose and fructose, for example, K, Ca, or Na. The fructose-containing solution and the purified D-psicose-containing solution may be obtained by the chromatography. The purified D-psicose-containing solution may be a solution containing D-psicose with a purity of 90% (w/w) or higher, for example, 95% (w/w) or higher. Specifically, the purity of D-psicose may be 90% (w/w) to 99% (w/w) or higher. The fructose-containing solution may be a solution containing fructose with purity of 70% (w/w) or higher. The separated

fructose-containing solution may be reused in the D-psicose epimerization reaction in the chromatography. The fructose is denatured without separation and separated by the chromatography, and is then reused in the D-psicose epimerization reaction to increase the total yield. Before the reuse, the cooling in a range of 25°C to 45°C, and 30°C to 40°C may be further included.

The D-psicose-containing solution of the present disclosure may be concentrated. For example, the obtained purified D-psicose-containing solution with purity of 95% (w/w) or higher is concentrated so that the concentration of D-psicose reaches 75 brix(%) or more, for example, 80 brix(%) or more. The concentration may be performed specifically at a temperature of 50°C to 80°C, for example, 55°C to 70°C. At this time, in the concentration, a low-temperature evaporator may be used to prevent the denaturation of D-psicose.

The preparation method for the D-psicose crystal of the present disclosure includes a first step of mixing a D-psicose-containing solution and ethanol. Accordingly, the D-psicose may be partially precipitated. Further, the addition of the seed and the cooling to be described below may particularly contribute to separation of the crystal with a high yield.

The mixing in the first step may be performed at 20°C to 60°C. The mixing in the first step may be performed at specifically 30°C to 60°C, more specifically 40°C to 60°C. At a temperature lower than this, the state becomes supersaturated, exceeding the metastable zone, and this causes the nucleation of new crystals rather than the growth of the crystal, and as a result, there is a problem in that the crystal growth is suppressed. At a higher temperature, the organic solvent is volatilized in the mixing process, and as a result, there is a problem in safety due to the decrease of the recovery rate and the generation of vapor.

The mixture of the D-psicose-containing solution and ethanol has a ratio of water : ethanol of 1:0.5 or higher, 1:0.7 or higher, or 1:1 or higher, and for example, 1:0.5 to 1:10, 1:0.7 to 1:10, 1:1 to 1:10, 1:1 to 1:9, or 1:1 to 1:8.

The preparation method for the D-psicose crystal of the present disclosure a second step of adding a seed to the mixed solution according to the first step and then cooling the same to obtain a massecuite containing the D-psicose crystal. Here, the massecuite means a slurry state in which the crystal and the solution are mixed when the D-psicose seed starts the crystallization reaction.

The most difficult aspect in the preparation method for the D-psicose crystal is controlling the size or the shape of the crystal. In the present disclosure, this may be achieved based on a crystallization method in which, after the organic solvent is mixed into the D-psicose-containing solution, the seed is added and the solution slowly cooled.

In the present disclosure, the D-psicose seed means a fine crystal mainly consisting of D-psicose, and it is added while uniformly maintaining a degree of supersaturation of the D-psicose in the organic solvent-mixed solution in the metastable zone. The organic solvent-mixed solution has low viscosity, and the added D-psicose seed may be easily dispersed. Under conditions of low viscosity and high dispersion, the growth of the crystal may be excellent. The D-psicose seed may be a seed crystal of 100 μm or less, and specifically a seed crystal having a size of 40 μm to 100 μm. The D-psicose seed may be added with a wt% of 0.01% (w/w) to 1% (w/w) based on the total weight of the mixed solution.

In the preparation method of the present disclosure, when the D-psicose seed is not added, an amorphous D-psicose lump is generated, and a D-psicose crystal having a desired size or shape is not generated. It is preferable that the D-psicose seed be added to the mixed solution in a dispersed form. After the addition of the seed, the crystal is grown by adjusting a cooling condition.

With respect to the cooling condition, the temperature in the first step of mixing before cooling is the same as described above, and a final temperature cooled according to the second step may be 8°C to 30°C, 8°C to 25°C, 8°C to 20°C, 10°C to 30°C, 10°C to 25°C, or 10°C to 20°C. According to such temperature control, the generation of new crystals is suppressed, thereby increasing the size of the crystal and increasing the yield. When the final temperature cooled according to the second step is higher than the above range, a recovery rate of the D-psicose crystal is lowered. In addition, when the final temperature is lower than the above range, the nucleation is induced, thereby generating a large amount of particles of less than 100 μm, resulting in aggregation between the crystals during drying, and as a result, a packaging volume of the final product is lowered or marketability is deteriorated.

In the present disclosure, the cooling rate is adjusted to control a growth rate of the D-psicose crystal. Specifically, the cooling may be performed at a rate of 0.05°C/hour to 1.4°C/hour, 0.6°C/hour to 1.4°C/hour, 0.7°C/hour to 1.3°C/hour, 0.8°C/hour to 1.2°C/hour, or 0.9°C/hour to 1.1°C/hour. As another example, the cooling rate may be adjusted to be cooled in the range of the metastable zone. When the cooling rate is faster than the range, the mixed solution rapidly enters a supersaturated zone beyond the metastable zone and causes a large amount of particles. Further, when the cooling rate is too low, productivity per unit time is reduced, resulting in inefficiency in mass production.

In the second step, the cooling and/or the crystallization may be performed for 20 hours to 70 hours or 30 hours to 70 hours.

According to an unlimited embodiment of the present disclosure, when the D-psicose crystal is prepared by using the organic solvent and controlling the cooling rate, a structured psicose crystal block may be generated.

The preparation method of the present disclosure may further include a third step of separating and drying the D-psicose crystal from the massecuite. Specifically, the D-psicose crystal containing an excess amount of organic solvent may be separated from the massecuite obtained by the first step and the second step.

In the separation of the D-psicose crystal from the massecuite, any method capable of separating the crystal may be used without limitation, but for example, a centrifugal dehydrator may be used. The separated crystal may contain 0.07% (w/w) or more, 0.1% (w/w) or more, and 0.13% (w/w) or more organic solvent, for example, 0.07% (w/w) to 0.5% (w/w), 0.1% (w/w) to 0.3% (w/w), or 0.13% (w/w) to 0.2% (w/w), based on 100% (w/w) of the total crystal.

The D-psicose crystal containing an excess amount of organic solvent is dried to obtain a D-psicose crystal containing 98% (w/w) or more D-psicose and 0.05% (w/w) or less ethanol based on 100% (w/w) of the total crystal. The description for the obtained D-psicose crystal is the same as above.

The preparation method of the present disclosure may further include a fourth step of recovering ethanol from a crystal mother liquor in which the D-psicose crystal is separated according to the third step, and then reusing the recovered ethanol in the first step.

Further, the preparation method of the present disclosure may further include a fifth step of reusing the crystal mother liquor in which ethanol is removed according to the fourth step in preparing a D-psicose solution in the first step.

The yield according to the preparation method of the present disclosure, that is, a weight percentage of the finally obtained D-psicose crystal compared to the weight of the D-psicose existing in the D-psicose-containing solution in the first step, may be 65% (w/w) or higher, 70% (w/w) or higher, 75% (w/w) or higher, or 80% (w/w) or higher.

That is, in the present disclosure, the yield may be represented by the following Formula.

Yield (%) = (weight of dehydrated and dried D-psicose crystal / weight of D-psicose in crude solution before crystallization) $\times$ 100 [Formula 1]

When the yield is calculated, the weight of D-psicose in the crude solution before crystallization is measured with D-psicose g/L in the crude solution through HPLC analysis, and then the measured D-psicose g/L is substituted according to a pre-measured crystallization crude solution amount (L) to calculate a weight (g) of D-psicose included in a specific crude solution amount (L).

Additionally, the mother liquor separated in the crystallization, that is, a supernatant dehydrated from the massecuite, may be reused in the first step of recovering the organic solvent by distillation and mixing the recovered organic solvent with the D-psicose solution. The solution containing D-psicose, in which the organic solvent is removed after the distillation, is cooled to 30°C and may be re-circulated in a column in which a strongly acidic cation-exchange resin is substituted with a hydrogen group and a weakly acidic anion-exchange resin is substituted with a hydroxyl group, re-circulated by continuous chromatography, or re-circulated in the first step. The mother liquor generated in the D-psicose crystallization may be a fraction containing D-psicose with a purity of 75% (w/w) or higher, 85% (w/w) or higher, or 95% (w/w) or higher.

## Advantageous Effects

In the preparation method for the D-psicose crystal of the present disclosure, by the crystallization method of mixing the organic solvent in the D-psicose-containing solution, adding the seed, and then slowly cooling the mixture, it is possible to increase the yield of the D-psicose crystal from the D-psicose solution and prepare the D-psicose crystal at a sufficient size and an appropriate shape for use in mass production with no bad taste/smell.

## Description of Drawings

FIG. 1 is a micrograph of a D-psicose crystal prepared in Example 1.
FIG. 2 is a particle size analysis result of the D-psicose crystal prepared in Example 1.
FIG. 3 is a particle size analysis result of a D-psicose crystal composition prepared in Example 2.
FIG. 4 is a diagram illustrating a shape of a D-psicose crystal cake prepared in Example 6.
FIG. 5A is a scanning electron micrograph (SEM) of a surface of the D-psicose crystal prepared in Example 1. FIG. 5B is a scanning electron micrograph (SEM) of a surface of a D-psicose crystal prepared in the Comparative Example.
FIG. 6 is a diagram illustrating a method for measuring a repose angle.

## Mode for Invention

Hereinafter, the present disclosure will be described in more detail with reference to the following Examples. However, the following Examples are merely illustrative of the present disclosure, and the scope of the present disclosure is not limited thereto.

**Example 1: Preparation of high-purity D-psicose crystal composition with purity of 98% (w/w) or higher and containing 0.05% (w/w) of less ethanol**

**(1) Production of low-purity D-psicose solution using microorganism**

A 50 brix(%) fructose solution (enzyme reaction substrate solution) with a purity of 95% (w/w) or higher was prepared. As in the epimerization enzyme reaction disclosed in Korea Patent Publication No. KR 10-2011-0035805 A (Korea Patent Application No. 10-2009-0118465), where a D-psicose epimerase separated from a strain of *Corynebacterium glutamicum* KCCM 11046P was immobilized to a sodium alginate carrier and filled into isomerization equipment (isomerization tower, HANJOO Machine Industrial Co., Ltd.), and then the prepared enzyme reaction substrate solution passed through a heat exchanger at a space velocity (SV) [flow rate (L)/time (Hr)/resin amount (L)] by raising to a temperature of 50°C at a rate of 5°C to 20°C per hour to obtain an epimerized D-psicose solution. At this time, the purity of the D-psicose was about 24% (w/w).

**(2) Purification of D-psicose solution**

The epimerized D-psicose solution was first cooled to a temperature of 30°C to 40°C at a rate of 5°C to 10°C per hour by the heat exchanger, and was then passed through a column in which a decolorant was filled to be decolored, and subsequently passed through a column in which a strongly acidic cation-exchange resin substituted with a hydrogen group and a weakly acidic anion-exchange resin substituted with a hydroxyl group were filled at SV 3 to be desalinized. The final ionic composition was adjusted to be 10 microsiemens or less per unit cm through the measurement of an electric conduction system, and the purity of the D-psicose in the desalinized enzyme reaction solution was maintained at 24% (w/w).

**(3) Separation of high-purity D-psicose solution using chromatography**

The ion-purified D-psicose-containing solution was added to a low-temperature evaporator (forced thin-film evaporator, WELCRON Hantec), concentrated to a concentration of 60 brix(%) for a short time of 10 minutes to 15 minutes at a temperature of 55°C to 70°C, and again cooled at a rate of 5°C to 25°C per hour through the heat exchanger to be separated into a purified D-psicose solution having a purity of D-psicose of 95% (w/w) or higher and a fructose-containing solution having a purity of fructose of 70% (w/w) or higher by continuous chromatography in a column in which a strongly acidic cation-exchange resin with a calcium active group attached was filled at 50°C to 60°C.

The fructose-containing solution having a purity of 70% (w/w) or higher separated by the continuous chromatography was recovered and cooled at a rate of 20°C to 30°C per hour to be re-circulated in an epimerization process of fructose at 30°C.

**(4) Crystallization by concentration, organic solvent treatment, and cooling of D-psicose solution**

The purified D-psicose solution having a purity of 95% (w/w) or higher separated by the continuous chromatography was concentrated at a temperature of 55°C to 70°C to be adjusted to a concentration of 85.0 brix(%). The concentrated D-psicose solution having a purity of 95% (w/w) or higher was rapidly cooled to a temperature of 40°C at a rate of 5°C to 20°C per hour through the heat exchanger, and then mixed with ethanol corresponding to a weight ratio of water:ethanol = 1:1.13 as compared to water content excluding solids.

An appropriate amount of a seed was added to the D-psicose solution mixed with the ethanol cooled to 40°C, and this was then cooled to a final temperature of 10°C at a cooling rate of 1°C per hour and crystallized for 30 hours to obtain a massecuite containing the D-psicose crystal.

The massecuite containing the D-psicose crystal was added to a high-speed centrifugal dehydrator and spun at 4,000 rpm for 10 minutes, and then a supernatant was discharged to obtain a D-psicose crystal containing an excess amount of ethanol. At this time, the residual supernatant was washed by spraying deionized water or ethanol, and the concentration of ethanol contained in the obtained D-psicose crystal was about 0.15% (w/w).

The recovered D-psicose crystal containing an excess amount of ethanol was moved to a fluidized bed dryer or a vacuum dryer and dried for 1 hour to 2 hours to remove an excess amount of ethanol and obtain a D-psicose crystal having a purity of 98% (w/w) or higher and containing 0.03% (w/w) ethanol. An amount of the D-psicose crystal obtained after drying was 2,252 g, which was recovered at a rate of about 81% as compared to 2,780 g existing in the D-psicose solution separated and concentrated by the continuous chromatography, and the crystal size was MA 336 (FIGS. 1 and 2).

Additionally, the mother liquor separated in the crystallization, that is, a supernatant dehydrated from the massecuite, may be reused in the step of recovering the ethanol by distillation and mixing the recovered ethanol with the D-psicose solution. The solution containing D-psicose in which the ethanol is removed after the distillation is cooled to

30°C and may be re-circulated in a column in which the strongly acidic cation-exchange resin substituted with a hydrogen group and the weakly acidic anion-exchange resin substituted with a hydroxyl group are filled, or re-circulated by continuous chromatography.

**Example 2: Preparation of high-purity D-psicose crystal composition with purity of 98% (w/w) or higher and containing 0.05% (w/w) of less ethanol**

In Example 1 above, except that a D-psicose-containing solution concentrated to 80.0 brix(%) was cooled to 50°C, ethanol corresponding to a weight ratio of water:ethanol = 1:9 as compared to water content excluding solids was mixed, a cooling rate was 0.5°C per hour, and a final temperature was up to 20°C for 60 hours, a high-purity D-psicose crystal composition having a purity of 98% (w/w) or higher and 0.05% (w/w) or less ethanol was prepared in the same manner as in Example 1.

An amount of the obtained D-psicose crystal having a purity of 98% (w/w) or higher and containing 0.05% (w/w) or less ethanol was 2,307 g, which was recovered at a rate of about 83% as compared to 2,780 g of initially dissolved D-psicose, and the crystal size was MA 241 (FIG. 3).

**Example 3: Preparation of D-psicose crystal composition having purity of 98% (w/w) or higher and containing 0.05% (w/w) ethanol**

In Example 1 above, except that a drying time was 30 minutes to 1 hour, a high-purity D-psicose crystal composition having a purity of 98% (w/w) or higher was prepared in the same manner as in Example 1.

The obtained D-psicose crystal contained 0.05% (w/w) ethanol, and the purity was 98% (w/w) or higher.

**Example 4: Preparation of D-psicose crystal composition having purity of 98% (w/w) or higher and containing 0.06% (w/w) ethanol**

In Example 1 above, except that a drying time was 10 minutes to 20 minutes, a high-purity D-psicose crystal composition having a purity of 98% (w/w) or higher was prepared in the same manner as in Example 1.

The obtained D-psicose crystal contained 0.06% (w/w) ethanol, and the purity was 98% (w/w) or higher.

**Example 5: Preparation of high-purity D-psicose crystal by changing type of mixed organic solvent**

In Example 1 above, except that methanol and isopropyl alcohol were mixed in the concentrated D-psicose-containing solution, a high-purity D-psicose crystal having a purity of 98% (w/w) or higher was prepared in the same manner as in Example 1.

When methanol was used, the yield of the obtained D-psicose crystal was 33%, and a mean particle size was MA 109.

When isopropyl alcohol was used, the yield of the obtained D-psicose crystal was 32%, and a mean particle size was MA 61.

**Example 6: Preparation of high-purity D-psicose crystal composition using organic solvent without controlling cooling rate**

In Example 2 above, except that ethanol corresponding to a weight ratio of water:ethanol = 1:4 as compared to water content excluding solids was mixed in the concentrated D-psicose-containing solution, and a seed was added and then crystallized for 30 hours of cooling at a final temperature of 20°C within 30 minutes to 1 hour without controlling the cooling rate, a high-purity D-psicose crystal composition having a purity of 98% (w/w) or higher and containing 0.05% (w/w) or less ethanol was prepared in the same manner as in Example 2.

An amount of the obtained D-psicose crystal was 1,056 g, which was recovered at a rate of about 38% as compared to initially dissolved D-psicose, and a generated D-psicose crystal block was 1,084 g and confirmed as having been recovered at a rate of 39%.

**Comparative Example: Preparation of high-purity D-psicose crystal by cooling crystallization method without using organic solvent**

In Example 2 above, except that the concentrated D-psicose-containing solution was cooled to an initial temperature of 40°C and then cooled to 20°C for 80 hours without mixing ethanol, a high-purity D-psicose crystal having a purity of 98% (w/w) or higher was prepared in the same manner as in Example 2.

The yield of the obtained D-psicose crystal was 53%, and a mean particle size was MA 374.

**Test Example 1: Confirmation of effect of removing residual ethanol**

The high-purity D-psicose crystals in Example 1 (containing 0.03% (w/w) or less residual ethanol), the Comparative Example, Example 4, and Example 3 were consumed by a subject in a predetermined amount in powder form, and then a 3-point test was performed to evaluate whether a difference was distinguished. Two test groups were arranged with three options, and a tester consumed the three options in sequence without knowing the arrangement order to select which had a different taste. The test was performed by 20 testers three times, and whether there was a difference was evaluated according to the correct-answer rate over the total number of tests. A D-psicose crystal and a residual ethanol concentration % (w/w) used in the evaluation were shown in Table 1, and an evaluation result was shown in Table 2. The evaluation was performed by counting the number of correct answers, comparing the total answer number and the correct answer number with a significance test table, and determining whether there was a statistical significance. When the test was performed 60 times, if the correct answer number was 27 or more, it was determined that there was a significant quality difference.

[Table 1]

| | Example 3 | Example 1 | Example 4 | Comparative Example |
|---|---|---|---|---|
| Residual ethanol concentration (%, w/w) | 0.05 | 0.03 | 0.06 | 0 |

[Table 2]

| Comparative group | | Number of testing times | Number of correct answers | Correct-answer rate | Test results |
|---|---|---|---|---|---|
| | | | | % | Presence/absence of difference |
| Comparative Example | Example 4 | 60 | 48 | 80.0 | Presence |
| Comparative Example | Example 3 | 60 | 22 | 36.7 | Absence |
| Comparative Example | Example 1 | 60 | 12 | 20.0 | Absence |
| Example 4 | Example 3 | 60 | 43 | 71.7 | Presence |
| Example 4 | Example 1 | 60 | 46 | 76.7 | Presence |
| Example 3 | Example 1 | 60 | 11 | 18.3 | Absence |

It was confirmed that there was no functional difference in the D-psicose crystals (Examples 1 and 3) containing 0.05% (w/w) or less residual ethanol as compared to a D-psicose crystal (Comparative Example) prepared by a method without using an organic solvent. From this, it can be seen that when the residual ethanol is adjusted below a predetermined concentration, a taste equivalent to that of the original D-psicose crystal is achieved.

**Test Example 2: Confirmation of effect of reducing bad taste/smell intensity by removal of residual ethanol**

In Test Example 1 above, the bad taste/smell intensity for the same test group (Table 1) used in the test was evaluated. 20 testers consumed the D-psicose crystal, and then the bad taste/smell intensity was expressed as a level (5 points indicated a case where the bad taste/smell intensity was maximal), and the evaluation results were shown in Table 3. It was confirmed that there was no difference in bad taste/smell in the D-psicose crystals (Examples 1 and 3) containing 0.05% (w/w) or less residual ethanol as compared to a D-psicose crystal (Comparative Example) prepared by a method without using an organic solvent. From this, it can be seen that when the residual ethanol is removed to be below a predetermined concentration, the bad taste/smell is equivalent to that of the original D-psicose crystal.

[Table 3]

| Comparative Example | Example 4 | Example 3 | Example 1 |
|---|---|---|---|
| 1.9±0.8 | 3.0 ± 0.4 | 2.0 ± 0.9 | 1.9 ± 0.7 |

**Test Example 3: Confirmation of smooth surface effect by containing ethanol**

The surfaces of high-purity D-psicose crystals in Example 1 (containing 0.03% (w/w) or less residual ethanol) and the Comparative Example were examined with SEM. The results were shown in FIG. 5.

It was confirmed that as compared to the D-psicose crystal (FIG. 5B) without addition of ethanol in the Comparative Example, the surface of the D-psicose crystal (FIG. 5A) with addition of ethanol in Example 1 was smooth. From this, it can be seen that the smoothness of the surface of the D-psicose crystal is increased by adding ethanol, and thus the surface thereof is lustrous or glossy.

**Test Example 4: Confirmation of effect of increasing crystal fluidity by containing ethanol**

**Test Example 4.1: Measurement of fluidity**

The fluidity of the D-psicose crystal (Example 1) containing 0.03% (w/w) or less residual ethanol was compared with that of the D-psicose crystal (Comparative Example) prepared by a method without using an organic solvent and evaluated. For evaluation of the fluidity, a fluidity meter was used.

[Table 4]

| | |
|---|---|
| Inlet diameter | 1.5 cm |
| Tray diameter | 5.0 cm |
| Inlet-tray spacing | 15 cm |
| Added amount | 30 g |

Table 4 above is a table showing measurement conditions of the fluidity meter.

Fluidity = [(weight of added powder - weight of tray residual powder) / weight of added powder $\times$ 100)]

As a resultant value calculated according to the formula increases, the fluidity of the crystal increases.

**Test Example 4.2: Measurement of repose angle**

Additionally, an effect of increasing fluidity by containing ethanol was confirmed by measuring a repose angle. The repose angle refers to a maximum inclined angle which may be deposited without flowing down when deposits that have not yet set are deposited on a slope, and when the repose angle is smaller, the fluidity of the crystal is larger (FIG. 6).

The repose angle was obtained by measuring an angle of the top surface of a composition with a graduator after a D-psicose crystal composition passed through a funnel installed on a horizontal surface at a predetermined speed.

[Table 5]

| | Fluidity | Repose angle |
|---|---|---|
| Example 1 | 59.7 | 35 |
| Comparative Example | 48 | 45 |

Table 5 above is a table showing measurement results of the fluidity and the repose angle. From Table 5, it was confirmed that in the case of D-psicose with ethanol added, the fluidity was increased, and as a result, it is suggested that since D-psicose containing ethanol has low viscosity, crystallization proceeds while the seed is dispersed, and thus it may afford a high yield and degree of crystallization.

## Claims

1. A D-psicose crystal comprising:
98% (w/w) or more D-psicose and 0.001% (w/w) to 0.05% (w/w) ethanol based on 100% (w/w) of the entire crystal.

2. The D-psicose crystal of claim 1, wherein a mean particle size (MA) is 200 μm or more.

3. A preparation method for a D-psicose crystal comprising:

a first step of mixing a D-psicose-containing solution and ethanol, wherein the mixture has a water : ethanol ratio of 1:0.5 or higher; and
a second step of adding a seed to the mixed solution according to the first step and then cooling the same to obtain a massecuite containing the D-psicose crystal.

4. The preparation method of claim 3, wherein the mixing in the first step is performed at 40°C to 60°C, and a final temperature cooled according to the second step is 10°C to 20°C.

5. The preparation method of claim 3, wherein in the second step, the seed is grown in a metastable zone by adjusting a cooling rate.

6. The preparation method of claim 5, wherein in the second step, the cooling rate is 0.05 °C/hour to 1.4°C/hour.

7. The preparation method of claim 3, wherein in the second step, the cooling and the crystallization are performed for 20 hours to 70 hours.

8. The preparation method of claim 3, wherein the D-psicose-containing solution contains 95% (w/w) or more D-psicose.

9. The preparation method of claim 3, wherein a weight percentage of a finally obtained D-psicose crystal compared to the D-psicose content existing in the D-psicose-containing solution in the first step is 65% (w/w) or more.

10. The preparation method of claim 3, wherein the water : ethanol ratio is from 1:0.5 to 1:10.

11. The preparation method of claim 3, wherein in the D-psicose-containing solution, a concentration of the D-psicose is 80 brix to 85 brix.

12. The preparation method of claim 3, further comprising: a third step of separating and drying the D-psicose crystal from the massecuite.

13. The preparation method of claim 12, further comprising: a fourth step of recovering ethanol from a crystal mother liquor in which the D-psicose crystal is separated according to the third step, and then reusing the recovered ethanol in the first step.

14. The preparation method of claim 12, further comprising: a fifth step of removing ethanol from a crystal mother liquor in which the D-psicose crystal is separated according to the third step, and then reusing the crystal mother liquor in preparing a D-psicose-containing solution.

15. The preparation method of claim 12, wherein a concentration of ethanol in the D-psicose crystal is adjusted to 0.05% (w/w) or less by the drying.

**Patentansprüche**

1. Ein D-Psicose-Kristall, umfassend: 98 % (Gew./Gew.) oder mehr D-Psicose und 0,001 % (Gew./Gew.) bis 0,05 % (Gew./Gew.) Ethanol, bezogen auf 100 % (Gew./Gew.) des gesamten Kristalls.

2. D-Psicose-Kristall nach Anspruch 1,

wobei die mittlere Partikelgröße (MA) 200 μm oder mehr beträgt.

3. Verfahren zur Herstellung eines D-Psicose-Kristalls, umfassend:

einen ersten Schritt des Mischens einer D-Psicose-haltigen Lösung und Ethanol, wobei die Mischung ein Wasser:Ethanol-Verhältnis von 1:0,5 oder höher aufweist; und
einen zweiten Schritt des Hinzufügens eines Samens zu der gemischten Lösung gemäß dem ersten Schritt und anschließenden Abkühlens derselben, um eine Maische zu erhalten, die den D-Psicose-Kristall enthält.

4. Herstellungsverfahren nach Anspruch 3,
wobei das Mischen im ersten Schritt bei 40 °C bis 60 °C durchgeführt wird und die gemäß dem zweiten Schritt gekühlte Endtemperatur 10 °C bis 20 °C beträgt.

5. Herstellungsverfahren nach Anspruch 3,
wobei im zweiten Schritt der Samen durch Einstellen einer Abkühlungsgeschwindigkeit in einer metastabilen Zone gezüchtet wird.

6. Herstellungsverfahren nach Anspruch 5,
wobei im zweiten Schritt die Abkühlungsgeschwindigkeit 0,05 °C/Stunde bis 1,4 °C/Stunde beträgt.

7. Herstellungsverfahren nach Anspruch 3,
wobei im zweiten Schritt die Abkühlung und die Kristallisation 20 bis 70 Stunden lang durchgeführt werden.

8. Herstellungsverfahren nach Anspruch 3,
wobei die D-Psicose-haltige Lösung 95 % (Gew./Gew.) oder mehr D-Psicose enthält.

9. Herstellungsverfahren nach Anspruch 3,
wobei der Gewichtsanteil des schließlich erhaltenen D-Psicose-Kristalls im Vergleich zum D-Psicose-Gehalt, der in der D-Psicose-haltigen Lösung im ersten Schritt vorhanden ist, 65 % (Gew./Gew.) oder mehr beträgt.

10. Herstellungsverfahren nach Anspruch 3,
wobei das Wasser:Ethanol-Verhältnis zwischen 1:0,5 und 1:10 liegt.

11. Herstellungsverfahren nach Anspruch 3,
wobei in der D-Psicose-haltigen Lösung die Konzentration der D-Psicose 80 bis 85 Brix beträgt.

12. Herstellungsverfahren nach Anspruch 3, das ferner umfasst:
einen dritten Schritt zum Abtrennen und Trocknen des D-Psicose-Kristalls aus der Maische.

13. Herstellungsverfahren nach Anspruch 12, das ferner umfasst:
einen vierten Schritt, bei dem Ethanol aus einer Kristallmutterlauge zurückgewonnen wird, in der der D-Psicose-Kristall gemäß dem dritten Schritt abgetrennt wurde, und bei dem das zurückgewonnene Ethanol dann im ersten Schritt wiederverwendet wird.

14. Herstellungsverfahren nach Anspruch 12, das ferner umfasst:
einen fünften Schritt des Entfernens von Ethanol aus einer Kristallmutterlauge, in der der D-Psicose-Kristall gemäß dem dritten Schritt abgetrennt wird, und anschließendes Wiederverwenden der Kristallmutterlauge bei der Herstellung einer D-Psicose-haltigen Lösung..

15. Herstellungsverfahren nach Anspruch 12,
wobei die Ethanolkonzentration im D-Psicose-Kristall durch Trocknen auf 0,05 % (Gew./Gew.) oder weniger eingestellt wird.

## Revendications

1. Cristal de D-psicose comprenant :
98% (m/m) ou plus de D-psicose et 0,001% (m/m) à 0,05% (m/m) d’éthanol rapporté à 100% (m/m) du cristal entier.

2. Cristal de D-psicose selon la revendication 1, où une taille moyenne de particule (MA) est 200 μm ou plus.

3. Procédé de préparation d'un cristal de D-psicose , comprenant:

   une première étape de mélange d'une solution contenant du D-psicose-et de l'éthanol, où le mélange présente un rapport eau : éthanol de 1:0,5 ou plus ; et
   une deuxième étape d'addition d'une graine à la solution de mélange selon la première étape puis de refroidissement du tout pour obtenir une masse cuite contenant le cristal de D-psicose.

4. Procédé de préparation selon la revendication 3, dans lequel le mélange à la première étape est effectué à une température de 40°C à 60°C, et la température finale de refroidissement selon la deuxième étape est de 10°C à 20°C.

5. Procédé de préparation selon la revendication 3, dans lequel, dans la deuxième étape, la graine est cultivée dans une zone métastable en ajustant la vitesse de refroidissement.

6. Procédé de préparation selon la revendication 5, dans lequel, dans la deuxième étape, la vitesse de refroidissement est de 0,05 °C/heure à 1,4 °C/heure.

7. Procédé de préparation selon la revendication 3, dans lequel, dans la deuxième étape, le refroidissement et la cristallisation sont effectués pendant 20 heures à 70 heures.

8. Procédé de préparation selon la revendication 3, dans lequel la solution contenant le D-psicose contient 95% (m/m) ou plus de D-psicose.

9. Procédé de préparation selon la revendication 3, dans lequel le pourcentage en masse du cristal de D-psicose finalement obtenu, comparé à la teneur en D-psicose présent dans la solution contenant le D-psicose à la première étape, est de 65% (m/m) ou plus.

10. Procédé de préparation selon la revendication 3, dans lequel le rapport eau : éthanol est de 1:0,5 à 1:10.

11. Procédé de préparation selon la revendication 3, dans lequel dans la solution contenant le D-psicose, la concentration en D-psicose est de 80 brix à 85 brix.

12. Procédé de préparation selon la revendication 3, qui comprend en outre : une troisième étape de séparation et de séchage du cristal de D-psicose à partir de la masse cuite.

13. Procédé de préparation selon la revendication 12, qui comprend en outre : une quatrième étape de récupération de l'éthanol à partir de la liqueur mère de cristal dans laquelle le cristal de D-psicose est séparé selon la troisième étape, puis de réutilisation de l'éthanol récupéré dans la première étape.

14. Procédé de préparation selon la revendication 12, qui comprend en outre : une cinquième étape d'élimination de l'éthanol de la liqueur mère de cristal dans laquelle le cristal de D-psicose est séparé selon la troisième étape, puis de réutilisation de la liqueur mère de cristal pour préparer une solution contenant du D-psicose.

15. Procédé de préparation selon la revendication 12, dans lequel la concentration en éthanol dans le cristal de D-psicose est ajustée à 0,05% (m/m) ou moins par séchage.

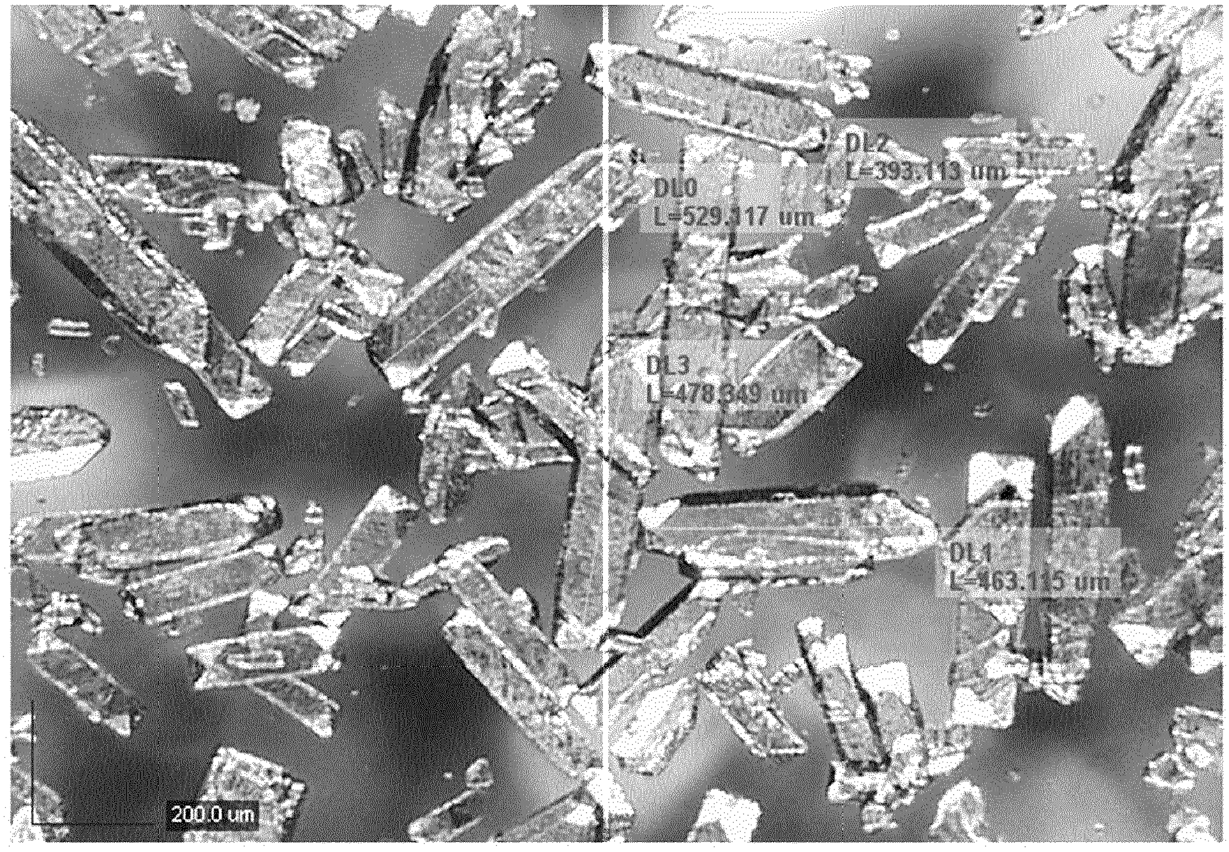
DL0
L=529.117 um
DL2
L=393.113 um
DL3
L=478.349 um
DL1
L=463.115 um
200.0 um

Fig.1

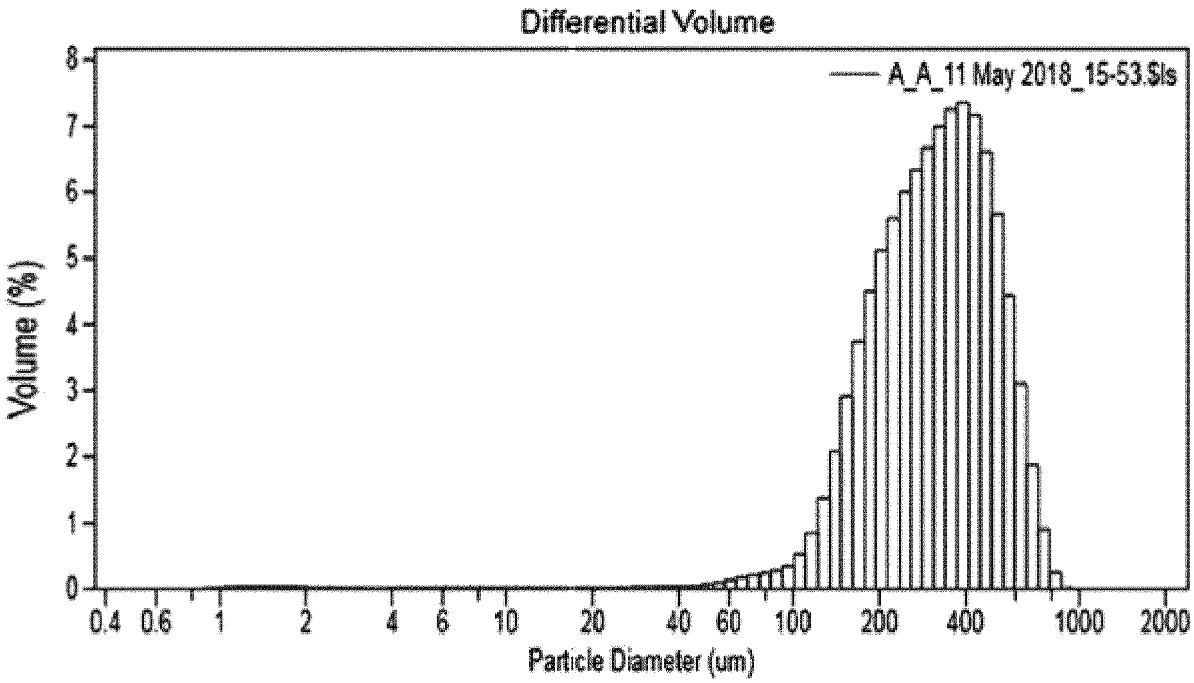


Volume Statistics (Arithmetic) A_A_11 May 2018_15-53.$ls

Calculations from 0.375 um to 2000 um

| | | | |
|---|---|---|---|
| Volume: | 100% | | |
| Mean: | 335.8 um | S.D.: | 152.4 um |
| Median: | 316.6 um | C.V.: | 45.4% |
| Mode: | 391.0 um | | |

$d_{10}$: 158.9 um $d_{50}$: 316.6 um $d_{90}$: 548.8 um

| <10% | <25% | <50% | <75% | <90% |
|---|---|---|---|---|
| 158.9 um | 217.7 um | 316.6 um | 437.7 um | 548.8 um |

**Fig.2**

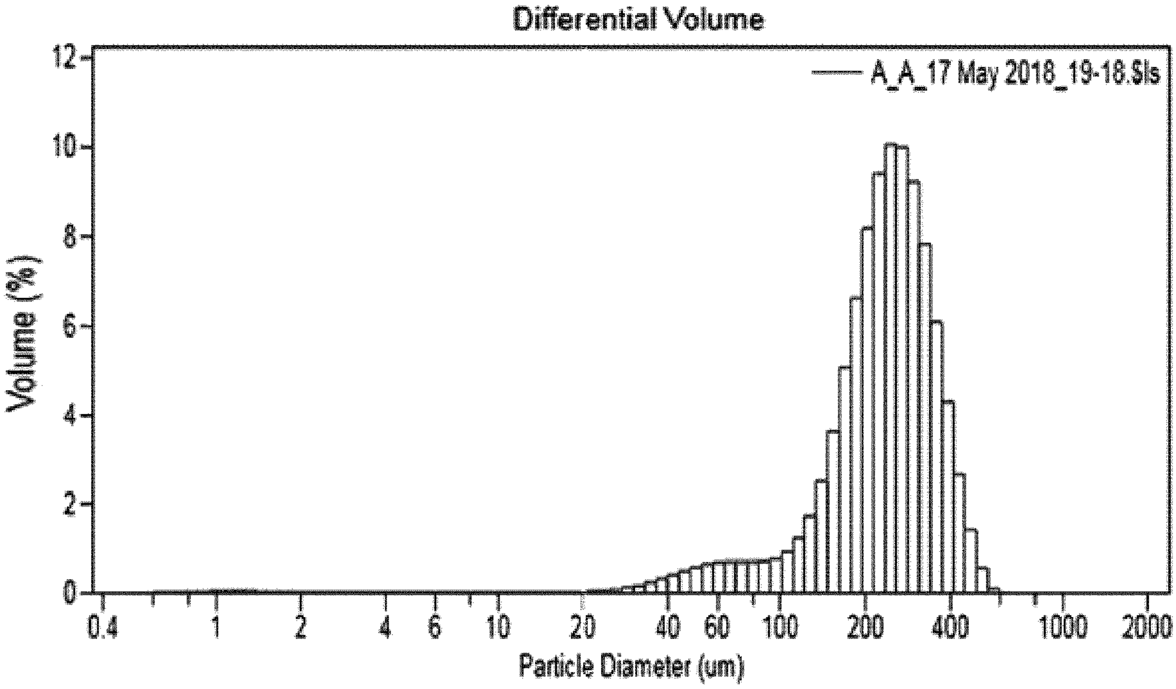


Volume Statistics (Arithmetic) A_A_17 May 2018_19-18.$ls

Calculations from 0.375 um to 2000 um

| | | | |
|---|---|---|---|
| Volume: | 100% | | |
| Mean: | 241.5 um | S.D.: | 97.88 um |
| Median: | 239.2 um | C.V.: | 40.5% |
| Mode: | 245.2 um | | |

$d_{10}$: 116.7 um $d_{50}$: 239.2 um $d_{90}$: 368.1 um

| <10% | <25% | <50% | <75% | <90% |
|---|---|---|---|---|
| 116.7 um | 180.8 um | 239.2 um | 303.6 um | 368.1 um |

**Fig.3**

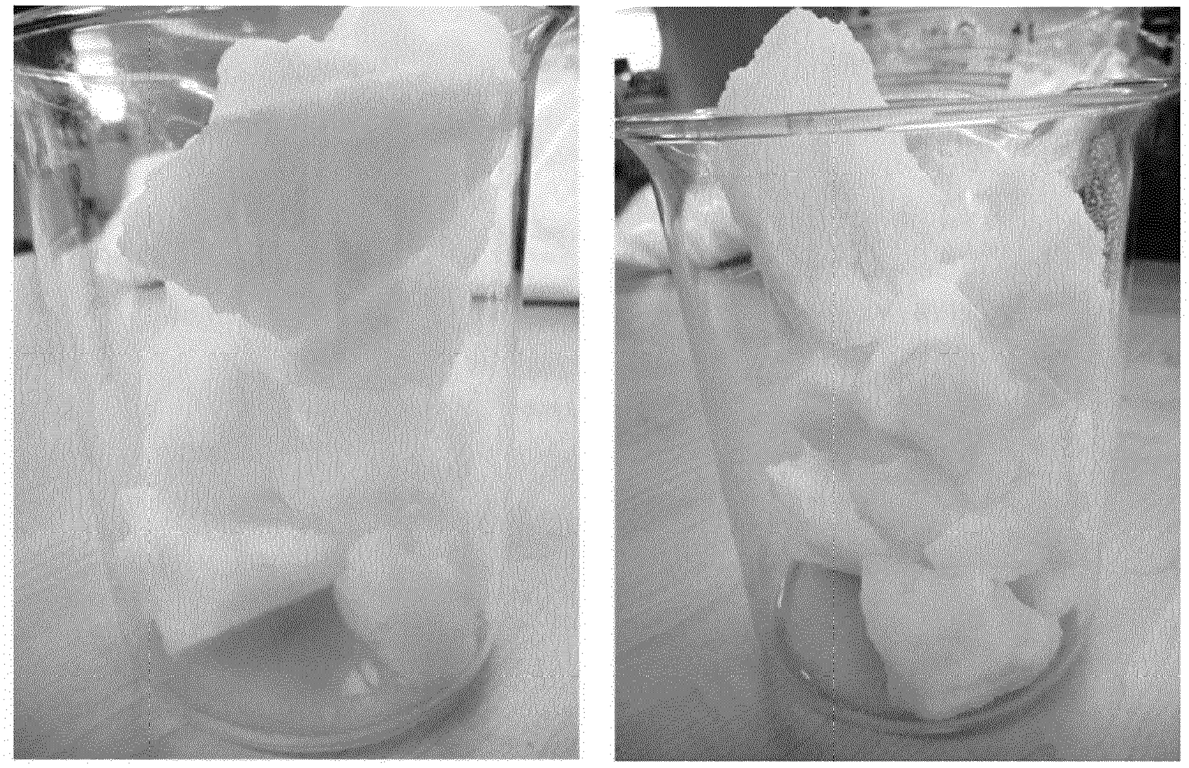

Fig.4

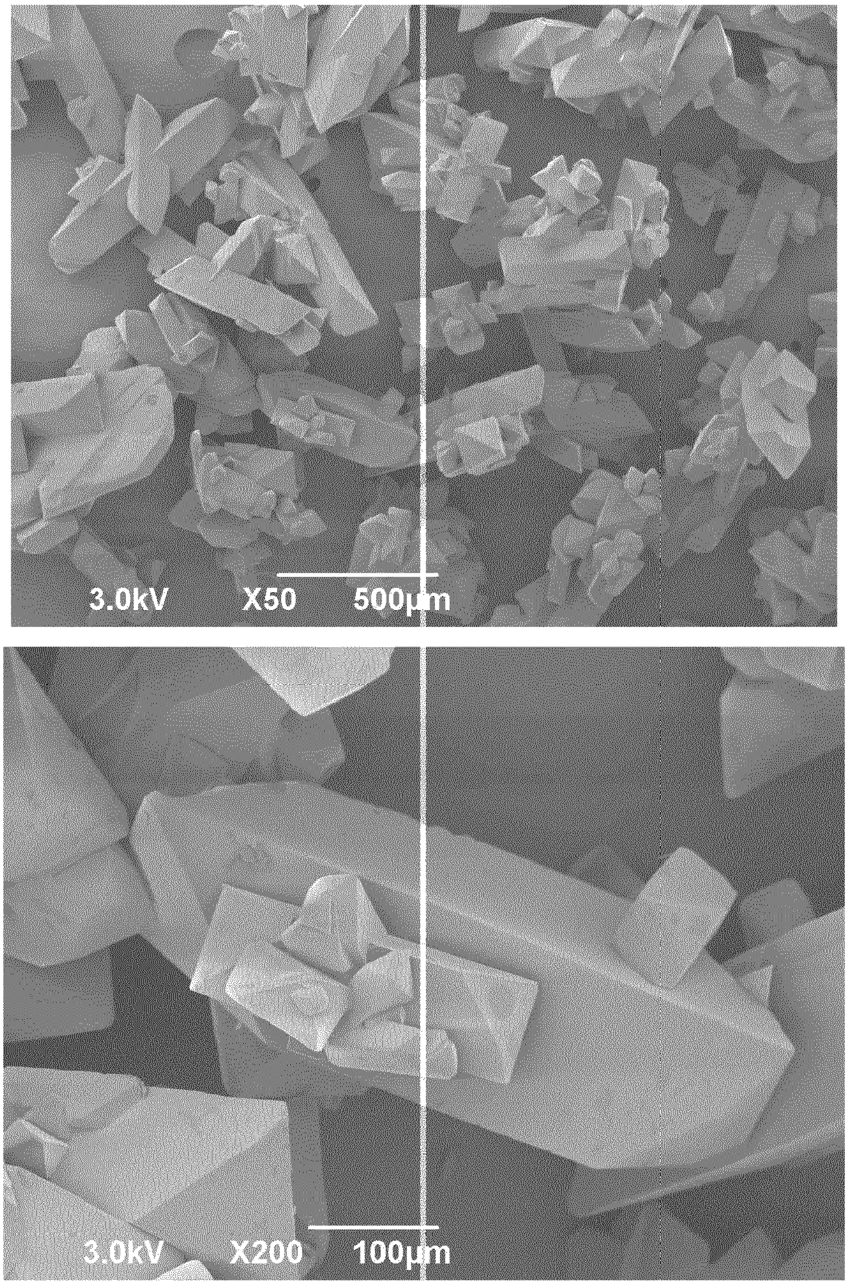


Fig.5A

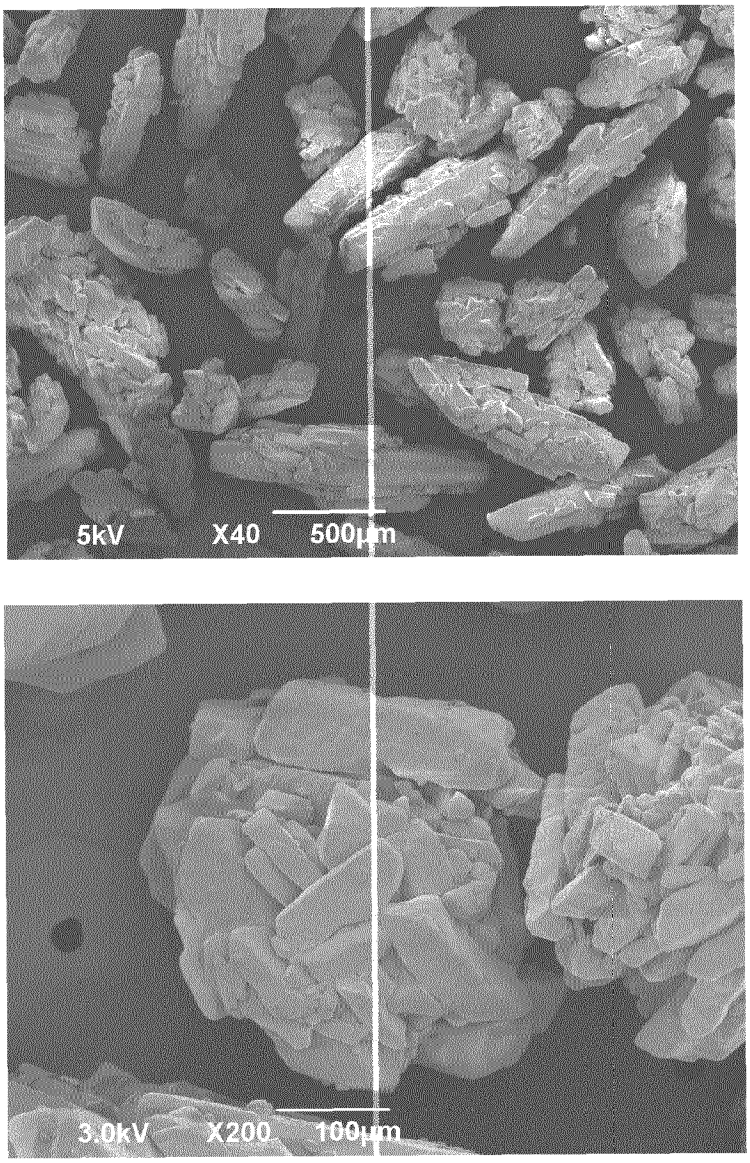


Fig.5B

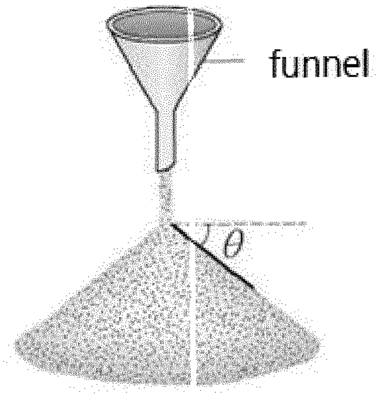


Fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3210478 A
- US 2011237790 A
- FR 3061413 A
- KR 102110035805
- KR 101203856
- KR 1020110035805
- KR 101455759
- KR 1020150047111
- KR 1020090118465


### Non-patent literature cited in the description


- **KEI T et al.** *J. Biosci. Bioeng.*, 2000, vol. 90 (4), 453-455